# EUROPEAN PATENT SPECIFICATION

(11) **EP 0 811 385 B1**
(45) Date of publication and mention of the grant of the patent: **27.08.2003**
(21) Application number: 97109034.5
(22) Date of filing: 04.06.1997
(51) Int. Cl.: A61K 47/48

(54) **Conjugate of a factor specifically recognizing interleukin-2 receptor with ribonuclease**
Konjugate von einem Interleukin-2 Rezeptor spezifisch erkennenden Faktor mit Ribonuklease
Conjugué d'un facteur reconnaissant spécifiquement le récepteur interleukin-2, avec la ribonucléase

(30) Priority: 05.06.1996 JP 14248596
(43) Date of publication of application: 10.12.1997
(73) Proprietor: Daiichi Suntory Pharma Co., Ltd., Tokyo (JP)
(72) Inventor: Ueda, Masakazu, Tokyo (JP)
(74) Representative: Kolb, Helga, Dr. Dipl.-Chem.

(56) References cited:
- EP-A- 0 466 816
- WO-A-94/15644
- CHEMICAL ABSTRACTS, vol. 125, no. 17, 21 October 1996 Columbus, Ohio, US; abstract no. 212093, JINNO, HIROMITSU ET AL: "Epidermal growth factor receptor-dependent cytotoxic effect by an EGF-ribonuclease conjugate on human cancer cell lines. A trial for less immunogenic chimeric toxin" XP002059565 & CANCER CHEMOTHER. PHARMACOL. (1996), 38(4), 303-308 CODEN: CCPHDZ;ISSN: 0344-5704, 1996,

## Description

The present invention relates to a conjugate of a factor specifically recognizing interleukin-2 receptor (hereinafter referred to simply as IL-2R) with a ribonuclease (hereinafter referred to simply as RNase) and a medicinal composition containing the same as the active ingredient.

Since only a few existing anticancer agents are efficacious against solid cancer and there appear multi drug-resistant cancer cells, it is urgently required to develop anticancer agents having novel function mechanisms. Moreover, studies have been aggressively made to establish drug delivery systems (DDS) for relieving side-effects and augmenting drug effects. However, no satisfactory result has been obtained so far.

To change this present situation, the present inventor has constructed conjugates of toxins or anticancer agents with monoclonal antibodies and revealed through experiments in vitro or in vivo that these conjugates might be used as remedies with benefits which are not recognized in the conventional ones (Hirota, N. et al., Cancer Res. 49:7106-7109, 1989). However, problems caused by foreign proteins contained in these conjugates arise. Namely, a protein conjugated of a toxin with a monoclonal antibody (i.e., an immunotoxin) is a foreign protein for humans and has some disadvantages such as, when administered to humans, it causes capillary leak syndrome as a side-effect and thus cannot be administered repeatedly over a long time.

Recently, it has been reported that bovine pancreatic RNase has remarkable anticancer effect and anti-HIV activity (Vescia, S. et al., Cancer Res. 40:3740-3744, 1980; Wu, Y.N. et al., J. Biol. Chem. 268:10686-10693, 1993; Nitta, K. et al., Cancer Res. 54:920-927, 1994; and Youle, R. et al., Proc. Natl. Acad. Sci. USA 91:6012-6016, 1994). These reports suggest that bovine RNase might be used for therapeutic purposes so long as it shows no antigenecity for humans. But so far as human RNase, no such an activity has been reported.

The present inventor has conducted studies to clarify whether or not human RNase widely occurring in various tissues and cells has a similar activity and consequently found that human RNase would be cytotoxic against human cells (Jinno, H. et al., Life Science 58(21):190-198, 1996). Since RNase is a physiologically active substance which exists in the human body, it has little antigenecity against humans. Further, the biological activities and behaviors of human RNase have been already clarified, which makes it possible to estimate the possible determine many side-effects accompanying the administration thereof. From these points of view, it is expected that human RNase might be highly advantageous as an anticancer drug. Thus, attempts have been made to develop a novel remedy specific for a certain tissue or cells by combining such cytotoxic RNase with a substance capable of specifically recognizing a certain tissue or cells.

In general, cancer cells overexpressing a growth factor receptor are highly malignant and thus can survive against conventional treatments. Accordingly, such cancer cells require to be treated by a therapy targeting the growth factor receptor expressed on the cell membrane (Ozawa, S. Cancer Res. 63:2169-2173, 19 ; Kitazawa, Y., Clin. Cancer Res. in press; and Ikeda, Y., Annal of Thoracic Surgery, in press). Recently, the present inventor has constructed a conjugate of human epithelial growth factor (EGF), which recognizes cancer cells overexpressing human epithelial growth factor receptors, with RNase as an anticancer agent being aimed at relieving side-effects and augmenting drug effect. As a result, the conjugate shows cytotoxicity depending on the EGF receptor (Jino, H. et al., Cancer Chemotherapy and Pharmacology 38:303-308, 1996).

However, there is a problem that the EGF-RNase conjugate can be produced only in a poor yield and thus not sufficiently available in practice. Further, the EFG-RNase conjugate is seemingly unusable in the treatment of immunological diseases, since EFG cannot specifically recognize cells in an immune system. Thus it is required to develop a conjugate which is effective in the treatment of immunological diseases also.

Under these circumstances, the object of the present invention is to produce a drug useful as an anticancer agent which has scarcely any antigenecity or side-effects, can be produced at a practically available yield and is usable in the treatment of immunological diseases also.

In order to achieve the above-mentioned object, the present inventor has conducted extensive studies. As a result, he has found that a conjugate of interleukin-2 (hereinafter referred to simply as IL-2) with RNase is cytotoxic for cells (cancer cells, etc.) overexpressing IL-2R, thus completing the present invention.

Accordingly, the present invention provides a conjugate of a factor specifically recognizing interleukin-2 receptor with a ribonuclease.

The present invention further provides a medicinal composition containing the above-mentioned conjugate as the active ingredient.

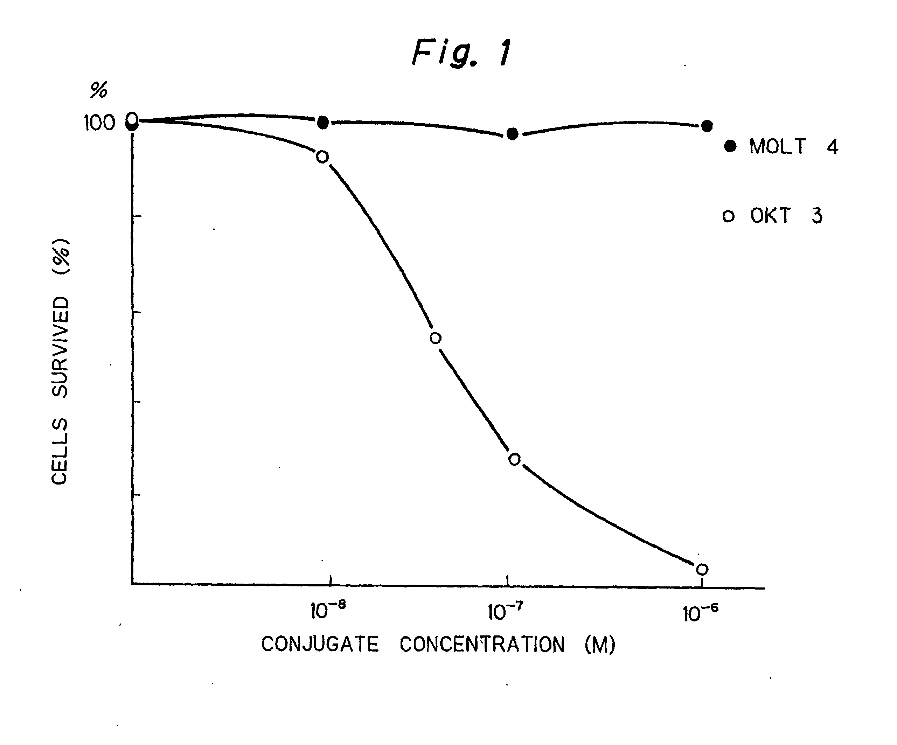

Fig. 1 is a graph showing the cytotoxicity of the conjugate of the present invention.

The term "a factor specifically recognizing IL-2R" as used herein means any substance having an affinity for IL-2R and thus involves IL-2, active fragments thereof, IL-2R antibodies, active fragments thereof, etc. That is to say, the factor specifically recognizing IL-2R to be used in the present invention may be an arbitrary one so long as it has an affinity for IL-2R. Thus all substances detected by, for example, the competitive assay with the use of ¹²⁵I-IL-2 [Shinseikagaku Jikken Koza (Lectures on New Biochemical Experiments) 12 "Bunshi Meneki-gaku (Molecular Immunology) I-Meneki Saibo, Saitokain (Immunocytes and Cytokines), p. 170-176, Tokyo Kagaku Dojin, 1989] fall within this category. IL-2R consists of α-, β- and γ-chains and all substances having affinities for each of these chains or combinations thereof are also involved therein. These factors specifically recognizing IL-2R may originate in natural matters. Alternatively, those produced by genetic engineering techniques may be used therefor.

Ribonuclease (RNase), which means any substance capable of degrading RNA, is also called RNA (ribonucleic acid) degradative enzyme. There are known several human RNases typified by RNase 1. The ribonuclease of the present invention involves not only these RNases but also modifications and variants thereof, so long as they are capable of degrading RNA. Examples of the RNase modifications include those wherein the binding moiety to an RNase inhibitor (for example, several amino acids located at the N-terminal of human RNase 1) has been deleted therefrom but the RNA degradation activity is still sustained. The RNase may originate in natural matters. Alteratively, those produced by genetic engineering techniques may be used therefor.

The conjugation of the factor specifically recognizing IL-2R with the RNase means that these substances are chemically linked to each other while allowing the exhibition of both effects. The conjugate can be constructed by various means commonly employed by those skilled in the art.

The conjugate can be constructed by a chemical synthesis method. For example, a factor specifically recognizing IL-2R such as IL-2 is modified with N-succinimidyl 3-(2-pyridyl-dithio)propionate (SPDP), etc. to thereby introduce thiol group thereinto. Separately, thiol group is introduced into the RNase by using 2-iminothiolane, etc. Then these substances are linked to each other via an S-S bond to thereby give the desired conjugate.

Also the conjugate of a factor specifically recognizing IL-2R with RNase according to the present invention can be constructed by a genetic engineering method. For example, a gene composed of a DNA encoding human IL-2 and another DNA encoding human RNase is integrated into a plasmid and then expressed in host cells capable of efficiently expressing this fused protein. It is thus possible to produce, for example, a protein in which the N-terminal of IL-2 has been linked to the C-terminal of the RNase at the gene level.

The cDNA encoding human IL-2 can be obtained from, for example, ATCC. As the cDNA encoding human RNase, on the other hand, use can be made of, for example, those obtained by the methods described in references (Seno, M., et al., Biochim. Biophys. Acta 1218:466-468, 1994; and Futami, J. et al., Biochem. Biophys. Res. Commun. 216:406-413, 1995).

The genetic engineering technique for producing the conjugate may be carried out in accordance with known procedures by using a polynucleotide encoding the amino acid sequence which is the same as that of the above-mentioned conjugate of the factor specifically recognizing IL-2R with RNase and optionally has partial deletion or substitution, or the above-mentioned amino acid which has an additional amino acid sequence consisting of one or more amino acids. Although the present invention is not particularly restricted, the expression efficiency can be regulated by, for example, adding or improving a signal sequence, selecting an appropriate host/vector system, or improving the expression-controlling site in the gene. It is also possible to obtain a conjugate having sugar chain(s) added thereto by selecting an appropriate host.

The fused protein thus obtained can be isolated and purified by procedures well known in the art. These procedures include centrifugation, ammonium sulfate salting out, various chromatographic techniques, etc., though the present invention is not restricted thereto.

The conjugate of the present invention can be processed into a medicinal composition by mixing with pharmacologically acceptable carriers, fillers, diluents, etc. The medicinal composition of the present invention is preferably administered by the methods commonly employed for peptide drugs, namely, parenteral administration such as intravenous administration, intramuscular administration or subcutaneous administration. When orally administered, the medicinal composition of the present invention is degraded in the digestive tracts. Thus, this administration route is not effective in general. However, it is possible to formulate a preparation for oral administration which is hardly degradable in the digestive tracts, for example, microcapsules having the conjugate of the present invention as the active ingredient enclosed in liposomes. Also, the conjugate of the present invention may be absorbed via mucosae other than the digestive tracts (for example, rectal, nasal or sublingual administration). In such a case, the conjugate can be administered in the form of suppositories, nasal sprays or sublingual tablets.

Although the dose of the medicinal composition of the present invention varies depending on the type of the disease, the age and body weight of the patient, the severity of symptoms, the administration route, etc., the dose may range generally from 0.1 µg/kg body weight to 100 mg/kg body weight, preferably from 0.5 µg/kg body weight to 5 mg/kg body weight and still preferably from 1 µg/kg body weight to 1 mg/kg body weight.

To examine the cytotoxic activity of the conjugate of the present invention, ATL (adult T cell leukemia) cells expressing IL-2R and control cells expressing no IL-2R are incubated in the presence of the conjugate. As a result, the conjugate of the present invention shows dose-dependent cytotoxicity for the ATL cells expressing IL-2R. In contrast, no cytotoxicity is observed for the control cells expressing no IL-2R. Thus it is clarified that the conjugate of the present invention exhibits cytotoxicity specifically for cells expressing IL-2R.

Human RNase usually occurs in the blood and has little immunogenicity. Therefore, it may be considered to be an excellent drug ingredient. It is suggested that the overexpression of IL-2R in some diseases such as adult T cell leukemia (ATL) might have some pathological relevance to these diseases. Thus the selective cytotoxicity of the conjugate of the present invention for cells overexpressing human IL-2R is applicable to the development of remedies for cancer such as leukemia, angiosarcoma, renal cancer and lymphoma, AIDS, adult type diabetes I, rheumatoid arthritis, spotted psoriasis, etc. and immunosuppressive agents to be used in, for example, graft rejection reaction in association with organ transplantation. In particular, the present invention has successfully established the first case of the production of remedies for the above-mentioned diseases which are composed exclusively of human physiologically active substances.

Because it is composed exclusively of human physiologically active substances, the conjugate of the present invention seemingly has minimal side-effects and thus can be administered over a long period of time. Owing to the factor specifically recognizing IL-2R, further, the conjugate selectively acts on tissues or cells specifically expressing IL-2R.

Cells overexpressing interleukin receptor (IL-R) are restricted to cancer cells such as adult T cell leukemia (ATL) cells, T cells undergoing immunopathy, etc. Therefore, drugs containing as the active ingredient the conjugate of the present invention of a factor specifically recognizing IL-2R with RNase are useful as selective anticancer agents, immunosuppressive agents to be used in, for example, graft rejection reaction in association with organ transplantation and remedies for AIDS, adult type diabetes I, articular rheumatism, spotted psoriasis, etc.

### EXAMPLES

To further illustrate the present invention in greater detail, and not by way of limitation, the following Referential Examples and Examples will be given.

### Referential Example 1:

### Extraction and purification of human RNase from liver tissue

Human liver was homogenized under acidic conditions of pH 4.0 and the solution thus obtained was allowed to stand at 4°C overnight. Then the homogenate was adjusted to pH 6.8 and centrifuged at a high speed. The supernatant thus obtained was adsorbed by a column packed with Cellulose Phosphay P11 (manufactured by Whatman) equilibrated with a 0.05 M phosphate buffer (pH 6.8) and eluted with linear gradiation of sodium chloride concentration. The eluate was subjected to gel filtration through a column packed with Sephadex G-150 (manufactured by Pharmacia) equilibrated with a 0.05 M Tris-hydrochloride buffer (pH 7.5), adsorbed by an affinity column packed with polyguanylic acid/agarose (manufactured by Sigma) equilibrated with the same buffer, and then eluted with a buffer containing 1 M of sodium chloride to thereby purify human RNase.

### Referential Example 2:

### Genetic engineering method for producing human RNase 1

Human pancreatic RNase 1 can be prepared by the genetic engineering method reported by J. Futami et al. (BBRC, 216:406-413, 1995). Briefly speaking, a cDNA insert of human RNase 1 (Seno, M. et al., Biochem. Biophys. Acta 1218:466-468, 1994) was inserted into plasmid pET3a. With the plasmid pBO26 thus obtained, Escherichia coli MM294(DE3)/pLysS(13) was transformed and the resulting transformant was incubated in LB-broth supplemented with glucose (0.4%), ampicillin (50 µg/ml) and chloramphenicol (10 µg/ml) until the absorbance at 600 nm reached 0.4. Subsequently, isopropyl 1-thio-β-D-galactopyranoside (IPTG) was added for induction and the incubation was continued at 37°C for additional 3 hours. Then the cells were harvested by centrifugation, washed with physiological saline and stored at - 80°C until they were used.

In the presence of 20% sucrose, the cells were disrupted by osmotic shock and subjected to freezing/thawing. The insoluble fraction containing the inclusion bodies of the expressed protein was solubilized. After refolding the protein, it was further purified by column chromatography to thereby give human RNase 1.

### Example 1:

### Construction of human IL-2-human RNase 1 conjugate (chemical synthesis method)

Construction was performed in accordance with the method of Newton, D.L. et al. (J. Biol. Chem. 267:19572, 1992). To modify IL-2, human IL-2 (human recombinant IL-2, manufactured by Sigma, USA) was reacted with N-succinimidyl 3-(2-pyridyl-dithio)propionate (SPDP) at 5 molar equivalent concentration of IL-2 at room temperature for 30 minutes to thereby introduce dithiol group into the IL-2. In this step, the unreacted SPDP was eliminated by passing the reaction mixture through a column packed with Sephadex G-25 (manufactured by Pharmacia) equilibrated with a 100 mM phosphate buffer (pH 7.5) containing 100 mM of sodium chloride.

On the other hand, thiol group was introduced into the human pancreatic RNase obtained in Referential Example 2 by using 2-iminothiolane (2-IT). Namely, RNase 1 was reacted with 2-IT at 5 molar equivalent concentration of RNase 1 at room temperature for 90 minutes. Thus RNase 1 was modified while the unreacted 2-IT was eliminated by using Sephadex G-25 in the same manner as the one described above.

The thus modified human IL-2 and human RNase 1 were mixed together at the equimolar ratio and reacted at 4°C for 12 hours.

The reaction product was purified by passing through a column packed with Sephadex G-50 (manufactured by Pharmacia) equilibrated with a 100 mM phosphate buffer (pH 7.5) and thus the IL-2-RNase conjugate of the present invention was obtained.

### Example 2:

### Construction of human IL-2-RNase 1 conjugate (genetic engineering method)

Plasmid pTCGF-11 in Escherichia coli MC1061 strain containing the human IL-2 gene cloned from human peripheral blood cells was acquired from ATCC (Proc. Natl. Acad. Sci. USA 81:2543-2547, 1984), while plasmid pET-30a(+) and E. coli host cells were purchased from Novagen.

pBO49 DNA containing the human pancreatic RNase 1 gene cloned from human pancreas was kindly provided by Dr. Masaharu Seno, Faculty of Engineering, Okayama University.

The IL-2-RNase 1 conjugate was constructed in the following manner.

### Transformation

1) A DNA insert encoding IL-2 was cut off from the plasmid pTCGF-11 with restriction enzymes EcoRI and BamHI. Similarly, a DNA insert encoding human pancreatic RNase 1 was cut off from pBO49 with restriction enzymes EcoRI and XbaI.
2) These DNA inserts were ligated into the plasmid pET-30a(+) so that the DNA encoding IL-2 was fused with the carboxy-terminal of the DNA encoding human pancreatic RNase 1, followed by cloning under control of bacteriophage T7 promoter.
3) An expression host containing a chromosomal copy of the T7 RNA polymerase gene (λDE3 lysogens) was transformed by the above-mentioned expression vector. As the expression host, use may be made of E. coli BL21(DE3), HMS174(DE3), BL21(DE3)pLysS, HMS174(DE3)pLysS, etc. An expression host appropriate for the purpose can be selected from among these strains differing from each other in expression level, target gene stabilization potential and protease content. In this Example, E. coli BL21(DE3) was employed.
4) The transformant was incubated at 37°C in LB medium supplemented with kanamycin until the absorbance at 600 nm reached 0.4 to 0.6 (for about 3 hours).
5) Expression was induced by adding IPTG at a final concentration of 1 mM and incubation was continued for an additional 3 hours.

### Bacteriolysis and isolation of inclusion bodies

1) Cells were harvested by centrifugation and resuspended in 40 ml of a cold lysis buffer (50 mM Tris-pH 8.0, 25% sucrose, 1 mM EDTA).
2) After adding a protease inhibitor (PMSF 0.5 M) and Lysozyme (0.1 mg/ml), the mixture was incubated on ice for 30 minutes and then frozen at - 20°C for at least 1 hour.
3) It was rapidly thawed in a water bath at room temperature.
4) The DNA was sheared by sonication and digestion with DNase 1.
5) The soluble fractions were washed away with a detergent mixture (0.2 M NaCl, 1% deoxycholic acid, 1% Nonidet P-40™) and the insoluble fraction was recovered by centrifugation.
6) E. coli cell membrane fragments were released by washing with a Triton/EDTA solution (1% Triton X-100, 1 mM EDTA) and the fraction of the inclusion bodies was recovered by centrifugation. These procedure were repeated twice or thrice.

### Solubilization of inclusion bodies and refolding of protein

1) The fraction of the inclusion bodies was suspended and homogenized in 40 ml of a mixture of 6 M guanidine-HCl with 0.1 M Tris-Cl (pH 8.4).
2) The soluble matters were removed by centrifugation.
3) The supernatant was purged with nitrogen gas so as to remove the air.
4) After adding 0.1 M of reduced glutathione, the pH value of the mixture was maintained alkaline with NaOH.
5) After purging with nitrogen gas, the tube was closed and incubation was carried out at 37°C for at least 90 minutes.
6) The mixture was diluted 20-fold with 10 mM Tris-Cl (pH 8.4), 0.5 mM PMDS and 0.5 M glutathion oxide supplemented with 1 mg/ml of Zwittergent™3-14 followed by refolding at room temperature overnight or longer.
7) The protein aggregates were removed by centrifugation.

### Recovery of renatured protein and final purification steps

1) Ammonium sulfate salting out
   Ammonium sulfate was added up to 44 to 48% saturation and then the high-molecular weight proteins thus precipitated were filtered off. Next, ammonium sulfate was added up to 60% saturation and the target protein (the conjugate protein of the present invention) was recovered by filtration and resuspended in PBS.
2) Affinity column chromatography
   The conjugate protein obtained in the above 1) was applied onto an affinity column of anti-IL-2-heparin-Sepharose equilibrated with PBS and 0.15 M NaCl. After thoroughly washing, the column was eluted with 2.0 M NaCl. The eluate thus obtained was dialyzed against PBS or desalted.

### Example 3:

### Cytotoxicity of IL-2-RNase conjugate for leukemic cells

The human IL-2-human RNase conjugate obtained in Example 1 was added in vitro at concentrations of 10⁻⁹ to 10⁻⁶ M to an adult T cell leukemic cell line OKT3 expressing much IL-2R and a negative control cell line MOLT4 (ATCC No. CRL1582). Two days after the addition, the cytotoxicity was examined. Fig. 2 shows the results thus obtained. The conjugate showed dose-dependent cytotoxicity for the cell line OKT3 but no cytotoxicity for the negative control cell line MOLT4.

## Claims

1. A conjugate of a factor specifically recognizing interleukin-2 receptor with a ribonuclease, wherein said factor specifically recognizing interleukin-2 receptor is selected from a group consisting of IL-2, active fragments thereof, IL-2 receptor antibodies and active fragments thereof.

2. A conjugate as claimed in claim 1, wherein said factor specifically recognizing interleukin-2 receptor is interleukin-2.

3. A conjugate as claimed in claim 1, wherein said ribonuclease is human pancreatic ribonuclease.

4. A medicinal composition containing as the active ingredient a conjugate as claimed in claims 1 to 3.

5. A medical composition as claimed in claim 4, for use in treating cancer.

6. A medicinal composition as claimed in claim 5, for use in treating leukemia.

7. A medicinal composition as claimed in claim 4, for use in treating an immune disease, AIDS, adult type diabetes I, articular rheumatism and spotted psoriasis.

8. Use of a conjugate according to claims 1 to 3 for the production of a medicament.

9. Use of a conjugate according to claim 8, for the production of a medicament for the treatment of cancer.

10. Use of a conjugate according to claim 9 for the production of a medicament for the treatment of leukemia.

11. Use of a conjugate according to claim 8 for the production of a medicament for the treatment of an immune disease, AIDS, adult type diabetes I, articular rheumatism and spotted psoriasis.

## Patentansprüche

1. Konjugat eines Faktors, der spezifisch den Interleukin-2-Rezeptor erkennt, mit einer Ribonuklease, wobei der Faktor, der spezifisch den Interleukin-2-Rezeptor erkennt, ausgewählt ist aus der Gruppe, bestehend aus Interleukin-2, aktiven Fragmenten davon, IL-2-Rezeptor-Antikörper und aktiven Fragmenten davon.

2. Konjugat gemäss Anspruch 1, wobei der Faktor, der spezifisch den Interleukin-2-Rezeptor erkennt, Interleukin-2 ist.

3. Konjugat gemäss Anspruch 1, wobei die Ribonuklease humane Pankreas-Ribonuklease ist.

4. Medizinische Zusammensetzung, enthaltend als Wirkstoff ein Konjugat gemäss einem der Ansprüche 1 bis 3.

5. Medizinische Zusammensetzung gemäss Anspruch 4 zur Verwendung bei der Behandlung von Krebs.

6. Medizinische Zusammensetzung gemäss Anspruch 5 zur Verwendung bei der Behandlung von Leukämie.

7. Medizinische Zusammensetzung gemäss Anspruch 4 zur Verwendung bei der Behandlung einer Immunerkrankung, AIDS, adulter Typ I-Diabetes, Gelenkrheumatismus und punktförmiger Psoriasis.

8. Verwendung eines Konjugats gemäss Ansprüchen 1 bis 3 zur Herstellung eines Medikaments.

9. Verwendung eines Konjugats gemäss Anspruch 8 zur Herstellung eines Medikaments zur Behandlung von Krebs.

10. Verwendung eines Konjugats gemäss Anspruch 9 zur Herstellung eines Medikaments zur Behandlung von Leukämie.

11. Verwendung eines Konjugats gemäss Anspruch 8 zur Herstellung eines Medikaments zur Behandlung einer Immunerkrankung, AIDS, adulter Typ I-Diabetes, Gelenkrheumatismus und punktförmiger Psoriasis.

## Revendications

1. Conjugué d'un facteur reconnaissant de manière spécifique le récepteur de l'interleukine-2 avec une ribonucléase, dans lequel ledit facteur reconnaissant de manière spécifique le récepteur de l'interleukine-2 est choisi dans le groupe constitué par IL-2, des fragments actifs de celle-ci, des anticorps dirigés contre le récepteur de IL-2 et des fragments actifs de ceux-ci.

2. Conjugué tel que revendiqué dans la revendication 1, dans lequel ledit facteur reconnaissant de manière spécifique le récepteur de l'interleukine-2 est l'interleukine-2.

3. Conjugué tel que revendiqué dans la revendication 1, dans lequel ladite ribonucléase est une ribonucléase pancréatique humaine.

4. Composition médicinale contenant en tant qu'ingrédient actif un conjugué tel que revendiqué dans les revendications 1 à 3.

5. Composition médicinale telle que revendiquée dans la revendication 4, pour utilisation dans le traitement d'un cancer.

6. Composition médicinale telle que revendiquée dans la revendication 5, pour utilisation dans le traitement d'une leucémie.

7. Composition médicinale telle que revendiquée dans la revendication 4, pour utilisation dans le traitement d'une maladie immune, d'un SIDA, de diabètes I de type adulte, d'un rhumatisme articulaire et d'un psoriasis à taches.

8. Utilisation d'un conjugué selon les revendications 1 à 3 pour la production d'un médicament.

9. Utilisation d'un conjugué selon la revendication 8, pour la production d'un médicament pour le traitement d'un cancer.

10. Utilisation d'un conjugué selon la revendication 9, pour la production d'un médicament pour le traitement d'une leucémie.

11. Utilisation d'un conjugué selon la revendication 8, pour la production d'un médicament pour le traitement d'une maladie immune, d'un SIDA, de diabètes I de type adulte, d'un rhumatisme articulaire et d'un psoriasis à taches.
